Europäisches Patentamt

European Patent Office

Office européen des brevets

(11) Publication number: **0 312 034**
**A2**

# (12) EUROPEAN PATENT APPLICATION

(21) Application number: 88116967.6

(22) Date of filing: 12.10.88

(51) Int. Cl.⁴: **G01N 27/26 , G01N 33/52 , G01N 31/22**

(30) Priority: **15.10.87 JP 260361/87**

(43) Date of publication of application:
**19.04.89 Bulletin 89/16**

(84) Designated Contracting States:
**DE GB SE**

(71) Applicant: **FUJI PHOTO FILM CO., LTD.**
**210 Nakanuma Minami Ashigara-shi**
**Kanagawa 250-01(JP)**

(72) Inventor: **Hiratsuka, Nobuo c/o Fuji Photo Film**
**Co., Ltd.**
**11-46, Senzui 3-chome**
**Asaka-shi Saitama(JP)**
Inventor: **Katsuyama, Harumi c/o Fuji Photo**
**Film Co., Ltd.**
**11-46, Senzui 3-chome**
**Asaka-shi Saitama(JP)**

(74) Representative: **Kraus, Walter, Dr. et al**
**Patentanwälte Kraus, Weisert & Partner**
**Thomas-Wimmer-Ring 15**
**D-8000 München 22(DE)**

(54) **Method of detecting analyte.**

(57) A method of detecting an analyte, which comprises

(1) allowing a sheet-shaped material wetted with an aqueous solvent, the sheet-shaped material containing plural analytes which ahve been separated from each other by the migration using electric force, into contact substantially uniformly with an element for detection having a microporous layer containing a detective reagent composition capable of producing a detectable change by at least one kind of the aforementioned analytes and capable of migrating in the presence of the aforementioned aqueous solvent in a substantially dry state of the element, and (2) detecting the above detectable change produced in the sheet-shaped material.

According to the method of the invention, an accurate, sharp and uniform migration pattern having a high color concentration can be obtained by a simple operation in a short time.

## METHOD OF DETECTING ANALYTE

### BACKGROUND OF THE INVENTION

#### Field of the Invention

This invention relates to a method of detecting or determining a substance or component to be analyzed (analyte) which has been separated electrically such as by electrophoresis, isoelectric focussing or the like. This invention also relates to a method of developing or amplifying an image or pattern of analyte(s) which has been separated electrically.

#### Description of the Prior Art

Various methods of detecting or determining an analyte by separating electrically are widely utilized in the fields of biochemistry and clinical assay where a biological component is separated and analyzed. In these methods, at least one kind of analytes is detected or determined by separating plural analytes having a molecular electric charge from each other in a thin layer or sheet-shaped support medium by applying an electric field, such as electrophoresis. Among them, there are the methods of detecting an analyte which comprise spotting a sample containing the analyte onto a thin layer or sheet-shaped support medium for electrophoresis (hereinafter referred to as electrophoretic support), wetting the electrophoretic support with an electroconductive aqueous pH buffer solution, conducting electrophoretic migration of the sample, and detecting the analyte in the electrophoretic support in a wet state. In the above methods, a solution of a reagent or a reagent composition reacting with the object analyte to be detected to produce a color or a solution of a dye dyeing the analyte selectively or nonselectively (Both solutions are collectively called coloring solution.) is allowed to react with the analyte in the electrophoretic support to form a color by immersing the electrophoretic support into the coloring solution, by spraying the coloring solution on the surface of the electrophoretic support or by allowing a porous sheet, such as filter paper, in which the coloring solution has been impregnated in contact with the surface of the electrophoretic support. Alternatively, the analyte(s) are dyed, and produced color image or color pattern is detected. All of the above detection methods contain a coloring or dyeing process using a solution, and the analytical operation is troublesome. Furthermore, since the analyte diffuses into the coloring solution, various troubles occur, such as fading in the color image or color pattern, decreasing in the color density, and becoming worse in the reproducibility of the color image or color pattern. As a result, the detecting or determinating accuracy becomes inferior.

A known method to simplify the operation is comprised of transferring a water-soluble coloring reagent suspended in a water-soluble binder to an electrophoretic support using a transfer apparatus. When the transfer apparatus is placed on the surface of the electrophoretic support, the binder dissolves, and the water-soluble coloring reagent diffuses into the electrophoretic support. The coloring reagent reacts with the analyte which has been separated to form a color or to dye the analyte.

Another known method is comprised of transferring an analyte such as nuleic acid from an electrophoretic support to a microporous polyamide (nylon) membrane in a wet state with an aqueous solvent by the electroblotting technique or the like, and detecting it. Since the microporous membrane employed in this method is nonselective, all analytes are transferred to the microporous membrane. Subsequently, the microporous membrane is treated with a coloring solution, and the coloring solution is allowed to react with the analytes transferred into the microporous membrane to form a color or to dye the analytes. Thus, the produced color image or color pattern is detected. The microporous membrane used in this method does not contain a detecting reagent.

Still another known method (disclosed in J. Histochem. Cytochem., vol. 32, pp 1265-1274, 1984) is comprised of allowing a cellulose ester microporous membrane impregnated with a water-soluble fluorescent material in contact uniformly with an electrophoretic support containing isozymes separated by electrophoretic migration in a wet state with an aqueous solvent, and used for the identification of the isozymes.

Since, in all of the above detecting methods to simplify the operation, the microporous membrane containing a detecting reagent is allowed to be in contact with the electrophoretic support containing analyte(s) separated by electrophoretic migration in a wet state with an aqueous solvent, the aforementioned problems, such as fading in the color image or color pattern, decreasing in the color denisty, and becoming worse in the reproducibility of the color image or color pattern, are still unsolved.

Another method disclosed in Japanese Patent KOKAI No. 62-156552 is comprised of allowing an element for detection composed of a detecting reagent layer laminated onto a transparent support in contact uniformly with an electrophoretic support containing an analyte separated by electrophoretic migration in the almost absence of water. The detecting reagent layer contains a detecting reagent producing a colorimetrically or fluorometrically detectable nondiffusible chemical species suspended in a water-insoluble binder polymer. Subsequently, the element for detection is separated, and the coloring or the like produced in the element for detection by the interaction between the reagent in the element for detection and the analyte is detected. In this method, since the element for detection is allowed in contact with the electrophoretic support in the almost absence of water, the reaction time becomes long, even though they are warmed in an incubator at about 37°C to 45°C. Moreover, since it is difficult that the element for detection is allowed in contact with the electrophoretic support uniformly over the whole surface, the color density of electrophoretic image or electrophoretic pattern is liable to become uneven.

## SUMMARY OF THE INVENTION

An object of the invention is to provide a method of detecting a separation image or pattern of plural analytes in a sheet-shaped material, such as an electrophoretic support, wetted with an aqueous solvent, the electrophoretic support containing the analytes which have be separated from each other by the migration using electric force, accurately by a simple operation in a short time.

Another object of the invention is to provide a method of developing or amplifying a separation image or pattern of plural analytes in a sheet-shaped material, such as an electrophoretic support, wetted with an aqueous solvent, the electrophoretic support containing the analytes which have been separated from each other by the migration using electric force, as an accurate, sharp and uniform image or pattern having a high color density by a simple opuration in a short time.

Such objects have been achieved by a method of detecting an analyte which has been electrically separated, which comprises (1) allowing a sheet-shaped material wetted with an aqueous solvent, the sheet-shaped material containing plural analytes which have been separated from each other by the migration using electric force, into contact substantially uniformly with an element for detection having a microporous layer containing a detective reagent composition capable of producing a detectable change by at least one kind of the aforementioned analytes and capable of migrating in the presence of the aforementioned aqueous solvent in a substantially dry state of the element, and (2) detecting the above detectable change produced in the sheet-shaped material.

## DETAILED DESCRIPTION OF THE INVENTION

The analyte is the material capable of migrating in the aqueous solvent by the action of electric field based upon its molecular change and capable of separating from other analytes by the migration. Examples of the analyte are various components in a biological fluid, such as various enzymes, isozymes, antigens, antibodies, proteins, peptides, hormones, nucleotides, other cellular components contained in blood, lymph, saliva, urine or the like, enzyme-labeled antigens and enzyme-labeled antibodies. The method of the invention is particularly effective for the separation, detection, identification, and determination of various proteins such as serum proteins, lipoproteins, glycoproteins and globulins, enzymes such as amylase, creatine kinase, lactate dehydrogenase, alkaline phosphatase, glucose-6-phosphate dehydrogenase, adenylate kinase, γ-glutamyltransferase and choline esterase, and isozymes such as amylases, creative kinases, lactate dehydrogenases and alkaline phosphatases.

The sheet-shaped material used in the method of the invention includes electrophoretic supports (thin layer or sheet-shaped support medium for electrophoresis) and membrane or sheet-shaped medium for electrofocusing. As such electrophoretic medium sheets, there are various commercial electrophoretic medium sheets and other known sheets disclosed in various references and patent specifications, such as the electrophoretic medium sheets composed of membrane filter described in U.S.P. 4,128,470 and Japanese Patent Application Nos. 62-97093 and 62-97094, and electrophoretic medium sheets composed of polysufone membrane filter described in Japanese Patent KOKAI No. 62-27006, the electrophoretic medium sheets and membranes composed of polyacrylamide aqueous gel described in Aoki et al., "Saishin Denkieido-Ho (The Newest Electrophoresis Method)", pp 370-415, Hirokawa-Shoten, Tokyo, 1978, "Electro-phoresis, vol. 2, pp 213-219, ibid, vol. 2, pp 220-228, U.S.P. 4,415,428, U.S.P. 4,189,370, EP 0 116 404A and the like, agarose aqueous gel electrophoretic medium membrane, agar aqueous gel electrophoretic medium membrane and starch aqueous gel electrophoretic medium membrane described in the above "Saishin Denkieido-Ho", and the like. Among them, the electrophoretic medium sheets composed of membrane filter is preferred.

The aqueous solvent is usually water. The content of the aqueous solvent in the membrane filter is to fill the voids, and it varies according to the kind, the size of void, the void content and the like of the membrane filter. In the case that the aqueous solvent is water, the amount is usually about 1.5 times to about 10 times as much as the dry weight of the membrane filter.

The element for detection is a sheet or membrane composed of the microporous layer. The above sheet or membrane may be laminated to a sheet-shaped support. The microporous layer may be either of nonfilbrous microporous layer or fibrous microporous layer.

The nonfibrous microporous layer may be isotropic porous layers and includes the membrane filters (blushed polymer layer) composed of cellulose ester such as cellulose acetate, polyamide such as nylon-6 or nylon-66, polycarbonate of bisphenol A or the like described in U.S.P. 1,421,341, U.S.P. 3,992,158 and the like, the polyolefin microporous membranes such as polyethylene microporous membrane and polypropylene microporous membrane described in "The 9th Plastic Film Kenkyu-Kai Koza Koen Yoshi-Shu (Plastic Film Seminar Course Lecture Summaries)" published by Kobunshi Gakkai (Polymer Society) on February 22, 1984, Membrana, Inc. Catalogue (published in July, 1982) and the like, the microporous membrane composed of polysulfone membrane filter described in Japanese Patent KOKAI No. 62-27006 and the like and the continuous microspaces-containing porous layers where polymer microbeads are joined so as to contact with each other at a point by using a polymer adhesive (three-dimensional lattice structure layer) described in U.S.P. 4,258,001. The void size of the nonfibrous porous layer is in the range from about 50 nm to about 50 $\mu$m, preferably from about 100 nm to about 5 $\mu$m, the void content is in the range from about 20 % to about 90 %, preferably about 40 % to about 85 %, and the thickness is in the range from about 10 $\mu$m to about 500 $\mu$m, preferably from about 20 $\mu$m to about 350 $\mu$m.

The fibrous microporous layer includes woven fabrics, knitted fabrics, organic polymer fiber pulp-cantaining paper and the filter paper thereof, glass fiber pulp-containing paper and the filter paper thereof, fibrous nonwoven fabric and the like. Preferable woven fabrics are the plain weaves made of spun yarn (twist yarn) described in U.S.P. 4,292,272, EP 0 162 301A and the like, and among the plain weave fabrics, broad cloth and poplin are preferable. A suitable thickness of the yarn of the woven fabrics is in the range from about 20 S to about 150 S, preferably from about 40 S to about 120 S, represented by cotton yarn number, and in the range from about 35 D to about 300 D, preferably from about 45 D to about 130 D, represented by silk deniers. The thickness of the woven fabrics is in the range from about 50 $\mu$m to about 500 $\mu$m, preferably from about 80 $\mu$m to about 350 $\mu$m, and the void content of the woven fabrics is in the range from about 40 % to about 90 %, preferably from about 50 % to about 85 %. Subsequently, preferable knitted fabrics are made of spun yarn (twist yarn) knitted by warp knitting described in EP 0 162 302A and EP 0 162, 301A, and among the fabrics made by warp knitting, tricot fabric, raschel fabric, milanese fabric and double tricot fabric are preferable. A suitable thickness of the yarn of the knitted fabrics is in the range from about 40 S to about 150 S, preferably from about 60 S to about 120 S, represented by cotton yarn number, in the range from about 35 D to about 130 D, preferably from about 45 D to about 90 D, represented by silk deniers, and in the range from about 20 to about 50 as the gauge number in the knitting process. The thickness of the knitted fabrics is in the range from about 50 $\mu$m to about 500 $\mu$m, preferably from about 80 $\mu$m to about 350 $\mu$m, and the void content of the knitted fabrics is in the range from about 40 % to about 90 %, preferably from about 50 % to about 85 %. Preferable organic polymer fiber pulp-containing papers are the papers made of the pulp of polyethylene fiber alone and the papers made of the mixture pulp of 30 to 70 % of polyethylene fiber and natural fiber such as cellulose fiber. The thickness of the papers is in the range from about 50 $\mu$m to about 500 $\mu$m, preferably from about 80 $\mu$m to about 350 $\mu$m, and the void content of the papers is in the range from about 20 % to about 80 %, preferably from about 50 % to about 70 %. The oils and fats adhered to the woven fabrics and knitted fabrics during manufacturing yarns and fabrics are preferably substantially removed by washing or the like.

The microporous layer may contain a known surfactant, a pH buffer composition and the like. By the incorporation of a surfactant, the aqueous solvent uniformly permeates from the sheet-shaped material to the element, and the detecting reagent composition in the element also uniformly permeates into the sheet-shaped material. Preferable surfactants are nonionic surfactants.

The microporous layer may be laminated onto the surface of a support to form a multilayer structure. The support may be transparent, translucent or opaque. The transparent support may be a known water-impermeable support. Examples of the known support are films, sheets and plates, having smooth surfaces, made of polyethylene terephthalate, polycarbonate of bisphenol A, polystyrene or a cellulose ester such as cellulose diacetate, cellulose triacetate or cellulose acetate propionate. The translucent support may be a film, a sheet or a plate, having smooth surfaces and being made translucent or semi-milky by suspending a small amount of titanium dioxide particulates, barius sulfate particulates or the like in the same polymer as the above transparent support. The opaque support may be a film, a sheet or a plate, having smooth surfaces and being made opaque and light-reflective or milky by suspending titanium dioxide particulates, barius sulfate particulates or the like in the same polymer as the above transparent support, a paper impregnated or coated with a polymer similar to the above transparent support, a ceramic sheet or a milky glass sheet. Preferred opaque supports are the light-reflective or milky support made of the same polymer as the above transparent support suspended with titanium dioxide particulates, barius sulfate particulates or the like. The thickness of the support is in the range from about 50 $\mu$m to about 1mm, preferably from about 80 $\mu$m to about 300 $\mu$m, irrespective of transparent, translucent and opaque. A known undercoating or subbing layer or a known adhesive layer may be provided on the surface of the support, if necessary, and thereby, the adhesive force to the microporous layer is adjusted, usually increased.

The detecting reagent composition can migrate in the presence of the aqueous solvent, and reacts with or acts on at least one kind of analytes to produce a detectable change. Such a detecting reagent composition includes the compositions containing a chromogenic compound and a coupler, the chromogenic compound being coupled with the coupler through oxidation by hydrogen peroxide in the presence of peroxidase to produce a quinoneimine dye, the compounds of water-soluble leuco dyes or self-oxidative color-forming chromogenic compounds oxidized by hydrogen peroxide in the presence of peroxidase to produce a dye, the color-forming reagent compositions containing a tetrazolium salt compound, the enzyme substrates water-soluble or dispersible in water bonded to a detectable compound such as a dye, the enzyme substrates being water-soluble or dispersible in water bonded to a compound or an atomic group reacting with a third compound to produce a detectable compound such as a dye, water-soluble dyes and other detecting reagent compositions.

As the chromogenic compound of the composition containing a chromogenic compound and a coupler, there are 4-aminoantipyrine (4-aminophenazone, i.e. 1-phenyl-2,3-dimethyl-4-amino-3-pyrazoline-5-one, described in Ann. Clin. Biochem., 6, 24-27 (1969) and its hydrochloride and 4-aminoantipyrine analogs and its salt such as, tri-substituted-4-amino-3-pyrazoline-5-one and its hydrochloride such as 1-(2,4,6-trichlorophenyl)-2,3-dimethyl-4-amino-3 pyrazoline-5-one and its hydrochloride disclosed in EP 0103901A. As the coupler, there are phenol, phenolsulfonic acids (including alkali metal salts and alkaline earth salts) such as 2-hydroxy-1-benzenesulfonic acid, 1-naphthol, 2-naphthol, dihydroxynaphthalenes such as 1,7-dihydroxynaphthalene, naphtholsulfonic acids (including alkali metal salts and alkaline earth salts) such as 1-hydroxy-2-naphthalenesulfonic acid, and other phenol derivatives and other naphthol derivatives disclosed in Ann. clin. Biochem., 6, 24-27 (1969), U.S.P. 3,886,045, U.S.P. 3,983,005, U.S.P. 4,292,272 and U.S.P. 4,291,121.

The leuco dye includes triarylimidazole leuco dyes and their acetates disclosed in U.S.P. 4,089,747 such as 2-(4-hydroxy-3,5-dimethoxyphenyl)-4,5-bis[4-(dimethylamino)phenyl]-imidazole, diarylimidazole leuco dyes and their acetates disclosed in EP 0 122 641A such as 2-(3,5-dimethoxy-4-hydroxyphenyl)-4[4-(dimethylamino)phenyl]-5-phenethylimidazole, diarylindolylimidazole leuco dyes and their salts disclosed in U.S.P. 4,721,670 such as 2-(2-phenyl-3-indolyl)-4,5-di[4-(dimethylamino)phenyl]imidazole.

As the color-forming reagent compositions containing a tetrazolium compound, there are the color-forming reagent compositions containing a dehydrogenase corresponding to the analyte, at least one kind of oxidized-form coenzymes, at least one kind of dye precursors and at least one kind of electron carriers. In the case of using this color-forming reagent composition, the layer containing the electron carrier is preferably separated from the layer containing the dye precursor. Other components may be incorporated in either of the above layers or another layer. By this composition, in many cases, the degradation of the reagent composition in the course of time is decreased and the measuring sensitivity is increased.

As the enzyme substrates being water-soluble or dispersible in water bonded to a detectable compound, there are dye-bound starches such as Blue Starch.

As the enzyme substrates being water-soluble or dispersible in water bonded to a compound or an atomic group reacting with a third compound to produce a detectable compound, there are coupler-bound starches such as the starch derivatives bounded with N-methylanilino groups described in U.S.P. 4,268,628.

As the water-soluble dyes, there are Bromocresol Green, Bromocresol Purple, Bromophenol Blue, Ponceau 3R, Fast Blue PR and the like described in Kawai et al, "Serurosu Aseteito Denkieido-Ho Ni Yoru Kessei Tanpaku Bunkaku (Serum Protein Fractionation by cellulose Acetate Electrophoresis)"

As the other detecting reagent compositions, there are ninhydrin color-forming reagent compositions, Schiff's dyeing compositions and the like.

In every detecting reagent composition, a known pH buffer composition may be incorporated in the microporous layer together with the detecting reagent composition for maintaining a pH value in the range suitable for the analyte or the enzyme reaction and/or the coloring reaction caused by the detecting reagent composition. The pH buffer composition may be selected from various pH buffer compositions known in the field of electrophoresis, clunical chemical assay or physiology. Moreover, a surfactant, preferably a nonionic surfactant, and other components may be incorporated, if necessary.

The aforementioned detecting reagent composition may be incorporated in the microporous layer by a known method, such as, immersing the microporous layer into an aqueous detecting reagent composition solution followed by drying, spraying an aqueous detecting reagent composition solution onto the microporous layer following by drying, applying an aqueous detecting reagent composition solution onto the microporous layer followed by drying or the like.

When the microporous layer is laminated to the support, the lamination may be carried out according to a known method such as laminating the microporous layer to the support through a hydrophilic polymer layer or a adhesive layer provided on the support described in U.S.P. 4,340,565, U.S.P. 4,337,222 and the like. When two or more microporous layers are laminated to the support, the lamination may be carried out according to the microporous bonding (or microporous adhesion) method described in EP 0 166 365A, EP 0 226 465A and the like.

The element for detection may further contain one or more layers known in the field of the integral multilayer analytical element, such as adhesive layer, light-reflecting layer, radiation-blocking layer, intermediate layer, filtering layer, barrier layer and the like. The support may be provided with a mark for the alignment of the sheet-shaped material, a scale as the criterion for migration image or migration pattern, or the like.

The method of detecting or forming an image or pattern of an analyte of the invention is carried out according to the following procedure.

A liquid sample containing analyte(s) is spotted onto the sheet-shaped material, and the analytes are allowed to migrate by acting a suitable electric field (or electric current) on the sheet-shaped material. In the case of electrophoresis, an electric field of a definite ionic strength is allowed to act on the analytes under a definite pH value. In the case of isoelectric focussing, the analyte molecules are separated from each other by allowing a pH gradient to act on each molecule based upon the isoelectric point of the molecule. In either case, the analyte molecules are separated by electrophoretic migration in the state that the sheet-shaped material is wetted with the aqueous solvent.

After the separation among the analytes proceeds to a suitable degree, the surface of the sheet-shaped material wetted with the aqueous solvent is allowed to be in contact substantially uniformly with the element for detection having the microporous layer containing the detecting reagent composition in a substantially dry state of the element. At that time, the element is usually put on the sheet-shaped material, or the sheet-shaped material may be put on the element to the contrary. The aqueous solvent in the sheet-shaped material permeates the surface then the inside of the element through the interface between the sheet-shaped material and the element being in contact with each other. The detecting reagent composition in the element for detection becomes movable by the aqueous solvent, and it migrates to the surface then the inside of the sheet-shaped material. The contact state is continued until the reaction between the analyte and the detecting reagent composition or the action of the detecting reagent composition on the analyte sufficiently proceeds and until a detectable change sufficiently occurs. During this time, the moved detecting reagent composition generates a detectable change such as coloring or dyeing corresponding to the separation image or the separation pattern of at least one kind of the analytes on the surface or on the inside of the sheet-shaped material, or makes visible the separation image or the separation pattern of the analyte(s). The contact time between the sheet-shaped material and the element varies according to the kind of the analyte, the kind of the detecting reagent composition, the concentration of the analyte and the concentration of the detecting reagent compositon, and in general, it is in the range of about 20 seconds to about 30 minutes, preferably about 30 seconds to about 20 minutes, at about 25°C to about 45°C, preferably about 30°C to about 40°C. Subsequently, the element for detection is detached. Since the aqueous solvent is present, the element can readily be detached from the sheet-shaped material without breaking it. When the element for detection is substantially transparent, the detachment of the element for detection is not essential, and the detectable change can be detected in the contact state of the element with the sheet-shaped material. However, it is preferable that the detectable change is detected after detaching the element from the sheet-shaped material.

Since the inside of the element for detection is wetted by the aqueous solvent during the contact, there is a possibility that the analytes diffuse from the sheet-shaped material to the element and in horizontal directions (the directions along the surface of the element). Therefore, fading of the migration image or the migration pattern, lowering of the color density and worsening of the reproducibility of the image or the pattern are a matter of concern. However, it was found that these problems substantially do not occure in the method of the invention where the element for detection in a dry state is allowed to be in contact with the sheet-shaped material.

Preferable detectable changes produced in the sheet-shaped material are, in general, photometrically or spectrophotometrically detectable changes. Such detectable changes may be detected by measuring color, fluorescence, chemical or biochemical luminescence or phosphorescence using a suitable spectrophotometer and a measuring method. The measuring method may be optical density measuring method (colorimetry), total light quantity measuring method, or image or pattern recognizing (discriminating) method. When the sheet-shaped material is transparent or translucent, the detectable change is measured by transmission photometry or reflection photometry. While, when the sheet-shaped material is opaque, the detectable change is measured by reflection photometry.

## EXAMPLES

### Example 1

#### Preparation of Element for Detection

An aqueous polyvinyl alcohol solution was applied to the surface of a colorless transparent polyethylene terephthalate film (support) having smooth surfaces and a thickness of 200 μm, and dried to form a polyvinyl alcohol layer about 5 μm thick. The polyvinyl alcohol layer was wetted with water almost uniformly, and a cellulose acetate membrane filter having a nominal pore size of 1.2 μm, a void content of 70 % and a thickness of 50 μm was laminated thereon as the microporous layer. Subsequently, 0.1 % aqueous Bromocresol Green (BCG) solution of pH 7.0 was applied to the microporous layer, and dried at 80° C for 10 minutes to obtain the element for detection.

#### Electrophoresis of Sample

A rectangular cellulose acetate microporous membrane electrophoretic support ("Separax", Fuji Photo Film Co., Ltd., trade name) having a size of 110 mm x 60 mm and a thickness of 140 μm was impregnated with 60 mM Veronal buffer solution of pH 8.6, and each 0.8 μl of human sera was applied to the membrane in a width of 10 mm at intervals of 5 mm by an applicator. A constant current of 0.8 mA per 10 mm in width was impressed on the electrophoretic support according to a conventional manner, and electrophoresis was carried out for 40 minutes.

#### Visualization of Migration Pattern and Evaluation

Immediately, the aforementioned element for detection was carefully superposed on the electrophoretic support so that air bubbles were not entered, and allowed to stand at room temperature of about 23° C for 10 minutes. Meanwhile, the migration patterns of the albumin and the globlin in the fractionated serum proteins were dyed by the BCG of the element for detection.

As a result of comparing the dyed serum protein patterns with the dyed results obtained by a conventional dyeing solution method, the patterns obtained by the method of the invention were superior to the conventional patterns in the sharpness of the patterns, no occurrence of the turbulence in the fractionated patterns and the accuracy of the patterns.

By this experiment, it became clear that the detection of a migration pattern can be conducted accurately in a simple operation by the method of the invention.

### Example 2

#### Preparation of Element for Detection

An aqueous polyvinyl alcohol solution was applied to the surface of a coloress transparent polyethylene terephthalate film having smooth surfaces and a thickness of 200 μm, and dried to form a polyvinyl alcohol layer about 5 μm thick. The polyvinyl alcohol layer was wetted with water almost uniformly, and a polysulfone membrane filter having a nominal pore size of 1.0 μm, a void content of 75 % and a thickness of 30 μm was laminated thereof as the microporous layer. Subsequently, the following aqueous solutin of the detecting reagent composition was applied to the microporous layer, and dried at 37° C for 10 minutes to obtain the element for detection.

Nicotinamide adenine dinucleotide        510 mM/m²

Phenazine methosulfate      9 mM/m²
3-(4´,5´-dimethyl-2-thiazolyl)-2.5-
diphenyltetrazolium bromide      135 mM/m²
Lithium lactate      10.8 mM/m²
Dissolved in 500 ml of 70 mM Tris buffer solution of pH 7.4

Electrophoresis of Sample

A rectangular cellulose acetate microporous membrane electrophoretic support ("Separax", Fuji Photo Film Co., Ltd., trade name) having a size of 220 mm x 60 mm and a thickness of 140 μm was impregnated with 60 mM Veronal buffer solution of pH 8.6, and each 0.8 μl of human sera was applied to the membrane in a width of 10 mm at intervals of 2 mm by an applicator. A constant current of 0.8 mA per 10 mm in width was impressed on the electrophoretic support according to a conventional manner, and electrophoresis was carried out for 45 minutes.

Visualization of Migration Pattern and Evaluation

Immediately, the aforementioned element for detection was carefully superposed on the electrophoretic support so that air bubbles were not entered, and allowed to stand at 37°C for 20 minutes in a dark room so as not to dry. Meanwhile, the migration pattern of lactate dehydrogenase (LDH) in the fractionated serum proteins was formed by the formazan dye produced from lithium lactate, a substrate of LDH, contained in the element for detection through a serial coupled enzyme reactions.

As a result of comparing the developed LDH pattern with the conventional coloring method using a treatment with an aqueous reagent composition solution, the pattern obtained by the method of the invention was superior to the conventional pattern in the sharpness of the pattern, no occurrence of the turbulence in the fructionated pattern and the accuracy of the pattern.

By this experiment, it became clear that the detection of a migration pattern can be conducted accurately in a simple operation by the method of the invention.

## Claims

1. A method of detecting an analyte, which comprises
① allowing a sheet-shaped material wetted with an aqueous solvent, the sheet-shaped material containing plural analytes which ahve been separated from each other by the migration using electric force, in contact substantially uniformly with an element for detection having a microporous layer containing a detecting reagent composition capable of producing a detectable change by at least one kind of the aforementioned analytes and capable of migrating in the presence of the aforementioned aqueous solvent in a substantially dry state of the element, and
② detecting the above detectable change produced in the sheet-shaped material.

2. The method of claim 1 wherein said detectable change is coloring or dyeing and said detecting reagent composition is a coloring or dyeing reagent composition.

3. The method of claim 1 wherein said microporous layer is laminated to the surface of a water-impermeable water-resistant support.

4. The method of claim 1, claim 2 or claim 3 which further comprises a process of detaching said element from said sheet-shaped material incorporated between the aforementioned process 1 and the aforementioned process 2.

5. The method of claim 1, claim 2 or claim 3 wherein the aforementioned process ② is carried out in a state that said sheet-shaped material is allowed to be in contact with said element.

6. The method of claim 1 wherein said sheet-shaped material is an electrophoresis support medium, and said migration using electric force is electrophoresis.

7. The method of claim 1 wherein said sheet-shaped material is an electrophoresis support medium, and said migration using electric force is isoelectric focussing.

8. The method of claim 1, claim 6 or claim 7 wherein said aqueous solvent is an aqueous buffer solution.

9. The method of claim 1 or claim 3 wherein said microporous layer is a nonfibrous microporous membrane filter, a woven fabric, a knitted fabric, an organic polymer fiber pulp-containing paper, a filter paper, a glass fiber pulp-containg paper, a glass fiber pulp-containing filter paper or a fibrous non-woven fabric.